(19) Europäisches Patentamt
European Patent Office
Office européen des brevets

(11) **EP 2 314 264 B1**

(12) **EUROPEAN PATENT SPECIFICATION**

(45) Date of publication and mention
of the grant of the patent:
**08.10.2014 Bulletin 2014/41**

(21) Application number: **09794340.1**

(22) Date of filing: **29.06.2009**

(51) Int Cl.:
*A61F 13/49* *(2006.01)*      *A61F 13/53* *(2006.01)*
*B32B 27/12* *(2006.01)*      *A61L 15/22* *(2006.01)*

(86) International application number:
**PCT/JP2009/061812**

(87) International publication number:
**WO 2010/004894 (14.01.2010 Gazette 2010/02)**

(54) **WATER-ABSORBENT SHEET COMPOSITION**

WASSERABSORBIERENDE BAHNZUSAMMENSETZUNG

COMPOSITION DE FEUILLE HYDROABSORBANTE

(84) Designated Contracting States:
**AT BE BG CH CY CZ DE DK EE ES FI FR GB GR
HR HU IE IS IT LI LT LU LV MC MK MT NL NO PL
PT RO SE SI SK TR**

(30) Priority: **11.07.2008 JP 2008182028**

(43) Date of publication of application:
**27.04.2011 Bulletin 2011/17**

(73) Proprietor: **SUMITOMO SEIKA CHEMICALS CO.,
LTD.
Kako-gun, Hyogo 675-0145 (JP)**

(72) Inventors:
• **TAKATORI, Junichi**
**Hyogo 672-8076 (JP)**
• **HANDA, Masayoshi**
**Hyogo 672-8076 (JP)**
• **FUKUDOME, Shinya**
**Hyogo 672-8076 (JP)**
• **MAEDA, Nobuhiro**
**Hyogo 672-8076 (JP)**

(74) Representative: **HOFFMANN EITLE
Patent- und Rechtsanwälte
Arabellastrasse 4
81925 München (DE)**

(56) References cited:
**WO-A2-2004/016425      JP-A- 2001 252 307
JP-B- 63 003 062         JP-U- 6 058 931
JP-U- 6 058 952          JP-U- 6 059 039
US-A1- 2002 115 969**

**Description**

TECHNICAL FIELD

The present invention relates to a water-absorbent sheet composition which can be used in the fields of hygienic materials and the like. More specifically, the present invention relates to a water-absorbent sheet composition which can be suitably used in absorbent articles, such as thin-style disposable diapers. Furthermore, the present invention relates to an absorbent article such as disposable diapers obtainable from the water-absorbent sheet composition.

BACKGROUND ART

Body liquid absorbent articles for use in hygienic materials such as disposable diapers have a structure in which an absorbent material for absorbing a liquid such as a body liquid is interposed between a flexible liquid-permeable surface sheet (top sheet) positioned on a side contacting a body and a liquid-impermeable backside sheet (back sheet) positioned on a side opposite to that contacting the body.

In recent years, there are increasing demands on thinning and lighter weights of hygienic materials, from the viewpoint of designs, commercial distributions, disposability, and the like. On the other hand, a method for thinning that is generally performed in a conventional hygienic material is, for example, a method of reducing a hydrophilic fiber such as pulp fiber, which serves to fix a water-absorbent resin in an absorbent material, and increasing the water-absorbent resin.

As described above, an absorbent material in which a water-absorbent resin is used in a large amount with a lowered proportion of a hydrophilic fiber is preferred in thinning, from the viewpoint of reducing bulky hydrophilic fibers while retaining a liquid. However, when a liquid distribution or diffusion upon actually using in a hygienic material such as disposable diapers is considered, there is a disadvantage that if a large amount of the water-absorbent resin is formed into a soft gel-like state by absorption, a so-called "gel-blocking phenomenon" takes place, whereby liquid diffusibility is markedly lowered and a liquid permeation rate of the absorbent material is slowed down. This "gel-blocking phenomenon" is a phenomenon in which especially when an absorbent material in which water-absorbent resins are highly densified absorbs a liquid, a water-absorbent resin existing near a surface layer absorbs the liquid to even more densify soft gel that forms near the surface layer, so that a liquid permeation into an internal of an absorbent material is inhibited, thereby making the internal of the water-absorbent resin incapable of efficiently absorbing the liquid. Further, an absorbent material having reduced hydrophilic fibers that contributes to shape retention is likely to undergo deformation of shape, due to twisting, tear or the like before or after the absorption of a liquid. The absorbent material that undergoes deformation of shape has markedly lowered liquid diffusibility, so that the permeation rate is slowed down and a liquid leakage occurs, without being able to exhibit the ability of the absorbent material. In order to avoid such phenomena and maintain the absorption properties of the absorbent material, a ratio of hydrophilic fibers and a water-absorbent resin is limited, thereby posing limitations on the reduction of hydrophilic fibers even to the thinning of hygienic materials.

In view of the above, conventionally, as a means of inhibiting "gel blocking phenomenon" which occurs when reducing hydrophilic fibers and using a water-absorbent resin in a large amount, for example, proposals such as a method using two kinds of water-absorbent resins having different water-absorbent capabilities (see Patent Publication 1), a method using a water-absorbent resin having a high surface cross-linking density (see Patent Publication 2), and the like have been made.

However, in these methods, the absorption properties as the absorbent materials in which water-absorbent resins are used in large amounts cannot be satisfied. In addition, there arise some problems that the water-absorbent resin is localized before use and subject to be mobile during use because hydrophilic fibers that play a role of fixing the water-absorbent resin are reduced, so that the "gel-blocking phenomenon" is more likely to take place.

In order to solve these problems, a method of immobilizing a water-absorbent resin to hydrophilic fibers has been studied. For example, proposals such as a method of allowing thermoplastic adhesive fibers to be contained in an absorbent material comprising a water-absorbent resin and a hydrophilic fiber, and heat-fusing the absorbent material (see Patent Publication 3), a method of immobilizing a water-absorbent resin and hydrophilic fibers with an emulsion adhesive, and a method of immobilizing a water-absorbent resin to a substrate with a hot melt adhesive (see Patent Publication 4), and the like have been made.

However, when a water-absorbent resin is immobilized with an adhesive according to the method as described above, the swelling of the water-absorbent resin is likely to be suppressed because the water-absorbent resin is bound to the adhesive. Especially when a water-absorbent resin and hydrophilic fibers and the like are immobilized with a thermoplastic adhesive or an emulsion, there arise some problems that the water absorbency inherently owned by the water-absorbent resin would not be sufficiently exhibited, and the "gel-blocking phenomenon" is likely to take place.

There is also a method of immobilizing a water-absorbent resin to a substrate without using an adhesive, which is, for example, a method of adhering water-absorbent polymer particles in the process of polymerization to a fibrous substrate to carry out polymerization on the fibrous substrate (see Patent Publication 5), a method of polymerizing a monomer aqueous composition containing acrylic acid and an acrylic acid salt as main components on a nonwoven fabric substrate by means of electron beam irradiation (see Patent Publication 6), and the like.

In these methods, while the fibrous substrate is penetrated into the polymer particles to be firmly adhered, there are some disadvantages that it is difficult to complete the polymerization reaction in the substrate, so that unreacted monomers and the like remain in the substrate in large amounts.

PRIOR ART PUBLICATIONS

PATENT PUBLICATIONS

Patent Publication 1: Japanese Patent Laid-Open No. 2001-252307
Patent Publication 2: Japanese Patent Laid-Open No. Hei-06-057010
Patent Publication 3: Japanese Patent Laid-Open No. Hei-10-118114
Patent Publication 4: Japanese Patent Laid-Open No. 2000-238161
Patent Publication 5: Japanese Patent Laid-Open No. 2003-11118
Patent Publication 6: Japanese Patent Laid-Open No. Hei-02-048944

WO 2004/016425 describes a sealing mat, which swells under the influence of a liquid and in which a material that is made of a superabsorber is disposed between two fleece layers, said material fixing the fleece layers to each other. The highly swelling and light sealing mat can be used for sealing purposes in structural and civil engineering, particularly for building tunnels.

US 2002/115969 relates to an ultra-thin absorbent sheet member 1a in which an absorbent polymer powder is adhered to one surface of a first nonwoven fabric by a hotmelt adhesive such that absorbent polymer powder present areas and absorbent polymer powder absent areas exist; the absorbent polymer powder absent areas are present at opposite widthwise ends of the ultra-thin absorbent sheet member and at least one position between the opposite ends; the absorbent polymer powder is bonded to the first nonwoven fabric by first hotmelt adhesive layers formed on an upper side of the first nonwoven fabric and on a lower side of the absorbent polymer powder and a second hotmelt adhesive layer formed to cover upper sides of the absorbent polymer powder present areas and the absorbent polymer powder absent areas; and the first hotmelt adhesive layer and the second hotmelt adhesive layer are both made of an aggregate of linear hotmelt adhesive pieces.

DISCLOSURE OF INVENTION

PROBLEMS TO BE SOLVED BY THE INVENTION

An object of the present invention is to provide a water-absorbent sheet composition which has sufficient absorbency without undergoing deformation of shape after liquid absorption, while being thin in style, so that the sheet composition can be suitably used as disposable diapers. Another object of the present invention is to provide an absorbent article, such as disposable diapers, obtainable from the water-absorbent sheet composition.

MEANS TO SOLVE THE PROBLEMS

The present invention relates to:

[1] A water-absorbent sheet composition comprising a structure in which a water-absorbent resin and a hot melt adhesive are sandwiched with two or more sheets of hydrophilic nonwoven fabrics, wherein the hydrophilic nonwoven fabrics have a basis weight of 25 $g/m^2$ or more, and wherein the water-absorbent resin is contained in an amount of from 200 to 400 $g/m^2$, and wherein the hot melt adhesive is contained in an amount of from 0.10 to 0.50 times that of the amount of the water-absorbent resin contained (mass basis), wherein the hydrophilic nonwoven fabric is made of rayon fibers or a mixture of two or more kind of fibers including rayon fibers, and wherein the water absorbent resin is uniformly immobilized on the hydrophilic nonwoven fabric.
[2] an absorbent article comprising the water-absorbent sheet composition of the above [1] sandwiched between a liquid-permeable sheet and a liquid-impermeable sheet.

EFFECTS OF THE INVENTION

The water-absorbent sheet composition of the present invention exhibits some excellent effects that the sheet composition has sufficient absorbency without undergoing deformation of shape after liquid absorption, while being thin in style. Therefore, the water-absorbent sheet composition of the present invention is used as an absorbent material for disposable diapers, whereby a hygienic material not only that is thin and has excellent external design, but also free from inconveniences such as liquid leakage can be provided. In addition, the water-absorbent sheet composition of the present invention can be used in the field of agriculture, the field of construction materials, and the like, in addition to the field of hygienic materials.

BRIEF DESCRIPTION OF THE DRAWINGS

[Figure 1] A schematic view showing an outline of the constitution of an apparatus for determining the water absorption capacity under load.
[Figure 2] A schematic view when a test liquid is supplied, in the property evaluation of the water-absorbent sheet composition.
[Figure 3] A schematic view when an amount of re-wet is measured, in the property evaluation of the water-absorbent sheet composition.
[Figure 4] A schematic view when diffusion length is measured, in the property evaluation of the water-absorbent sheet composition.

BEST MODE FOR CARRYING OUT THE INVENTION

The water-absorbent sheet composition has a structure in which a water-absorbent resin and a hot melt adhesive are sandwiched with two or more sheets of hydrophilic nonwoven fabrics, and a thin-style water-absorbent sheet composition that does not contain hydrophilic fibers such as pulp fibers can be realized by adjusting an amount of the water-absorbent resin and an amount of the hot melt adhesive based on the water-absorbent resin. Further, in the water-absorbent sheet composition of the present invention, since a water-absorbent resin is immobilized to a nonwoven fabric with a hot melt adhesive, the localization or scattering of the water-absorbent resin can be inhibited, even though the sheet composition does not contain hydrophilic fibers such as pulp fibers, and the deformation in shape is also inhibited. In addition, an entire surface of the water-absorbent resin is not in the state that is covered with a hot melt adhesive, but a part is in the state of being immobilized; therefore, it is considered that the water-absorbent resin can be sufficiently swollen, without inhibiting water absorbency of the water-absorbent resin.
The water-absorbent sheet composition of the present invention may be an embodiment where hydrophilic fibers such as pulp fibers are admixed with the water-absorbent resin between the nonwoven fabrics in an amount within the range so as not to impair the effects of the present invention. An embodiment where substantially no hydrophilic fibers are contained is preferred, from the viewpoint of thinning.
As the kinds of the water-absorbent resins, commercially available water-absorbent resins can be used. For example, the water absorbent resin includes hydrolysates of starch-acrylonitrile graft copolymers, neutralized products of starch-acrylic acid graft polymers, saponified products of vinyl acetate-acrylic acid ester copolymers, partially neutralized products of polyacrylic acid, and the like. Among them, the partially neutralized products of polyacrylic acids are preferred, from the viewpoint of amount of production, production costs, water absorbency, and the like.
The partially neutralized product of a polyacrylic acid has a degree of neutralization of preferably 50% by mol or more, and even more preferably from 70 to 90% by mol, from the viewpoint of increasing osmotic pressure of the water-absorbent resin, thereby increasing water absorbency.
The water-absorbent resin is contained in an amount of from 200 to 400 $g/m^2$, preferably from 220 to 370 $g/m^2$, and more preferably from 240 to 330 $g/m^2$, from the viewpoint of obtaining sufficient absorbency even when the water-absorbent sheet composition of the present invention is used as disposable diapers or the like. When the water-absorbent resin is contained in an amount of less than 200 $g/m^2$, sufficient absorbency cannot be obtained as an absorbent material, so that an amount of re-wet is likely to increase. When the water-absorbent resin is contained in an amount exceeding 400 $g/m^2$, the gel-blocking phenomenon is more likely to take place, so that diffusion property of the liquid is worsened as an absorbent material, and the permeation rate is likely to be slowed down.
The absorbency of the water-absorbent sheet composition of the present invention is influenced by the water absorbency of the water-absorbent resin used. Therefore, it is preferable that the water-absorbent resin to be used in the present invention is those selected with optimal ranges in water absorption properties such as water absorption capacity (water-retention capacity), water absorption capacity under load, and water absorption rate of the water-absorbent resin, by taking the constitution of each component of the water-absorbent sheet composition or the like into consideration.

The water-absorbent resin has a water-retention capacity of saline solution of preferably 25 g/g or more, more preferably from 25 to 60 g/g, and even more preferably from 30 to 50 g/g, from the viewpoint of absorbing a liquid in a larger amount, and preventing the gel blocking phenomenon while keeping the gel strong during absorption. The water-retention capacity of saline solution of the water-absorbent resin is a value obtainable by a measurement method described in Examples set forth below.

In addition, taking into consideration of a case where the water-absorbent sheet composition of the present invention is used in disposable diapers, water absorption capacity under load is also important, from the viewpoint that the one with a higher water-absorption capacity is preferred even in the state where a sheet composition is exposed to a load of an individual who puts on the sheet composition. The water-absorbent resin has a water-absorption capacity of saline solution under load of 4.14 kPa is preferably 15 mL/g or more, more preferably from 20 to 35 mL/g. The water-absorption capacity under load of 4.14 kPa of the water-absorbent resin is a value obtainable by a measurement method described in Examples set forth below.

The water-absorbent resin has a water-absorption rate of saline solution of preferably less than 60 s, more preferably less than 50 s, from the viewpoint of speeding up the permeation rate of the water-absorbent sheet composition of the present invention and preventing a liquid leakage upon use in a hygienic material. The water-absorption rate of the water-absorbent resin is a value obtainable by a measurement method described in Examples set forth below.

The water-absorbent resin has a mass-average particle size of preferably from 50 to 1000 μm, more preferably from 100 to 800 μm, and even more preferably from 200 to 500 μm, from the viewpoint of preventing the scattering of the water-absorbent resin , the gel blocking phenomenon during water absorption and water absorption of the water-absorbent resin in the water-absorbent sheet composition, and at the same time reducing the rugged feel of the water-absorbent sheet composition, thereby improving texture.

The hot melt adhesive used in the present invention includes, for example, rubber adhesives such as natural rubbers, butyl rubbers, and polyisoprene; styrenic elastomer adhesives such as styrene-isoprene block copolymers (SIS), styrene-butadiene block copolymers (SBS), styrene-isobutylene block copolymers (SIBS), and styrene-ethylene-butylene-styrene block copolymers (SEBS); ethylene-vinyl acetate copolymer (EVA) adhesives; ethylene-acrylic acid derivative copolymer adhesives such as ethylene-ethyl acrylate copolymer (EEA), and ethylene-butyl acrylate copolymer (EBA); ethylene-acrylic acid copolymer (EAA) adhesives; polyamide adhesives such as copolymer nylons and dimer acids-based polyamides; polyolefin adhesives such as polyethylenes, polypropylenes, atactic polypropylenes, and copolymeric polyolefins; polyester adhesives such as polyethylene terephthalate (PET), polybutylene terephthalate (PBT), and copolymeric polyesters; and acrylic adhesives, and these adhesives may be used together in two or more kinds. In the present invention, the ethylene-vinyl acetate copolymer adhesives and the styrerlic elastomer adhesives are preferred, from the viewpoint of high adhesive strength, thereby making it possible to prevent exfoliation of a hydrophilic nonwoven fabric and scattering of the water-absorbent resin.

The hot melt adhesive has a melting temperature or a softening point of preferably from 60° to 180°C, from the viewpoint of sufficiently fixing a water-absorbent resin to a nonwoven fabric, and at the same time preventing thermal deterioration or deformation of the nonwoven fabric. Here, in the water-absorbent sheet composition of the present invention, in the process of producing a water-absorbent sheet composition, after melting, the hot melt adhesive is adhered to a nonwoven fabric or a water-absorbent resin in a solid state by cooling the molten adhesive.

The hot melt adhesive is contained in an amount of from 0.10 to 0.50 times, preferably from 0.12 to 0.30 times, and more preferably 0.15 to 0.25 times the amount of the water-absorbent resin contained (mass basis). When the hot melt adhesive is contained in an amount of less than 0.10 times, adhesion becomes insufficient, thereby making likely to cause exfoliation of the hydrophilic nonwoven fabrics themselves and increase in localization or scattering of the water-absorbent resin. When the hot melt adhesive is contained in an amount exceeding 0.50 times, the adhesion of the hydrophilic nonwoven fabrics is too strong to each other, and the swelling of the water-absorbent resin after water absorption is inhibited, thereby making it likely to lower water absorbency and a liquid retention capacity.

The water-absorbent sheet composition of the present invention also has a feature in the aspect in the use of two or more sheets of hydrophilic nonwoven fabrics. In general, when a water-absorbent sheet composition is used in a hygienic material, it is deduced that properties as the hygienic material such as permeation rate or amount of re-wet are greatly influenced by various properties such as hydrophilicity or strength of a nonwoven fabric on a liquid-permeable side (front side) in the water-absorbent sheet composition. In addition, in a case of a nonwoven fabric on a reverse side, it is deduced that the properties other than strength or flexibility are not as necessary as in the front side. However, during the present study, when only a nonwoven fabric of a reverse side is made hydrophobic, although the reasons therefor are not clear, a significantly large amount of liquid leakage is caused between the nonwoven fabrics during water absorption, so that it is found that a nonwoven fabric on a reverse side is also obliged to be hydrophilic. In other words, in the present invention, a thin-style water-absorbent sheet composition having high absorbency can be obtained by using hydrophilic nonwoven fabrics on both the sides of the nonwoven fabrics sandwiching the water-absorbent resin.

The hydrophilic nonwoven fabric used in the present invention is made of rayon fibers or a mixture of two or more kind of fibers including rayon fibers. The fabric may include polyolefin fibers such as polyethylene (PE) and polypropylene (PP); polyester fibers such as polyethylene terephthalate (PET), polytrimethylene terephthalate (PTT), and polyethylene naphthalate (PEN); polyamide fibers such as nylon; and other synthetic fibers. In addition, its surface may be subjected to a hydrophilic treatment according to a known method, as occasion demands. From the viewpoint of increasing the strength of the water-absorbent sheet composition, rayon fibers, optionally in admixture with polyolefin fibers and/or polyester fibers, are preferred. The hydrophilic nonwoven fabric made of synthetic fibers may contain pulp fibers in a small amount to an extent that the thickness of the water-absorbent sheet composition would not increase.

The hydrophilic nonwoven fabric is preferably a nonwoven fabric having an appropriate bulkiness and a large basis weight, from the viewpoint of giving the water-absorbent sheet composition of the present invention excellent liquid permeability, flexibility, strength and cushioning property, and speeding up the permeation rate of the water-absorbent sheet composition. The hydrophilic nonwoven fabric has a basis weight of 25 $g/m^2$ or more, preferably from 35 to 250 $g/m^2$, and more preferably from 50 to 150 $g/m^2$.

The water-absorbent sheet composition of the present invention can be produced, for example, by uniformly dispersing mixed powders of a water-absorbent resin and a hot melt adhesive on a hydrophilic nonwoven fabric, further overlaying a hydrophilic nonwoven fabric over the hydrophilic nonwoven fabric, and heat-and-pressing the overlaid fabrics at a temperature near a melting temperature of the hot melt adhesive.

In addition, the water-absorbent sheet composition of the present invention may properly be formulated with an additive such as a deodorant, a bactericidal agent, or a gel stabilizer.

The water-absorbent sheet composition of the present invention has a great feature in the aspect of enabling thinning of the composition. When the use in disposable diapers is taken into consideration, the water-absorbent sheet has a thickness, on a dry basis, of preferably 5 mm or less, more preferably 4 mm or less, even more preferably 3 mm or less, and still even more preferably from 0.5 to 2 mm.

The water-absorbent sheet composition of the present invention, when the use in disposable diapers is taken into consideration, has a water absorption capacity of saline solution of preferably 2,000 $g/m^2$ or more, more preferably 4,000 $g/m^2$ or more, and even more preferably from 6,000 to 18,000 $g/m^2$, from the viewpoint that the larger the absorption capacity the more favorable.

Further, the absorbent article of the present invention has a structure in which a water-absorbent sheet composition of the present invention is sandwiched between a liquid-permeable sheet and a liquid-impermeable sheet. The absorbent article includes, for example, disposable diapers, incontinence pads, sanitary napkins, pet sheets, drip sheets for foods, water blocking materials for electric power cables, and the like. Further, as the liquid-permeable sheet and the liquid-impermeable sheet, known ones in the technical field of the absorbent articles can be used without particular limitations. The absorbent article can be produced by a known method.

## EXAMPLES

The present invention will be specifically described hereinbelow by the Examples, without intending to limit the scope of the present invention thereto.

The properties of the water-absorbent resin and the water-absorbent sheet composition were measured in accordance with the following methods.

< Water-Retention Capacity of Saline Solution of Water-Absorbent Resin > The amount 2.0 g of water-absorbent resin was weighed in a cotton bag (Cottonbroad No. 60, width 100 mm x length 200 mm), and placed in a 500 mL-beaker. Physiological saline (0.9% by mass aqueous solution of sodium chloride, hereinafter referred to the same) was poured into the cotton bag in an amount of 500 g at one time, and the physiological saline was dispersed so as not to generate an unswollen lump of the water-absorbent resin. The upper part of the cotton bag was tied up with a rubber band, and the cotton bag was allowed to stand for 1 hour, to sufficiently swell the water-absorbent resin. The cotton bag was dehydrated for 1 minute with a dehydrator (manufactured by Kokusan Enshinki Co., Ltd., product number: H-122) set to have a centrifugal force of 167G. The mass Wa (g) of the cotton bag containing swollen gels after the dehydration was measured. The same procedures were carried out without adding water-absorbent resin, and the empty mass Wb (g) of the cotton bag upon wetting was measured. The water-retention capacity of saline solution of the water-absorbent resin was calculated from the following formula.

$$\text{Water-Retention Capacity of Saline Solution (g/g) of}$$

$$\text{Water-Absorbent Resin}$$

$$= [\text{Wa} - \text{Wb}] \text{ (g)/Mass (g) of Water-Absorbent Resin}$$

< Water-Absorption Capacity of Saline Solution of Water-Absorbent Resin Under Load of 4.14 kPa >

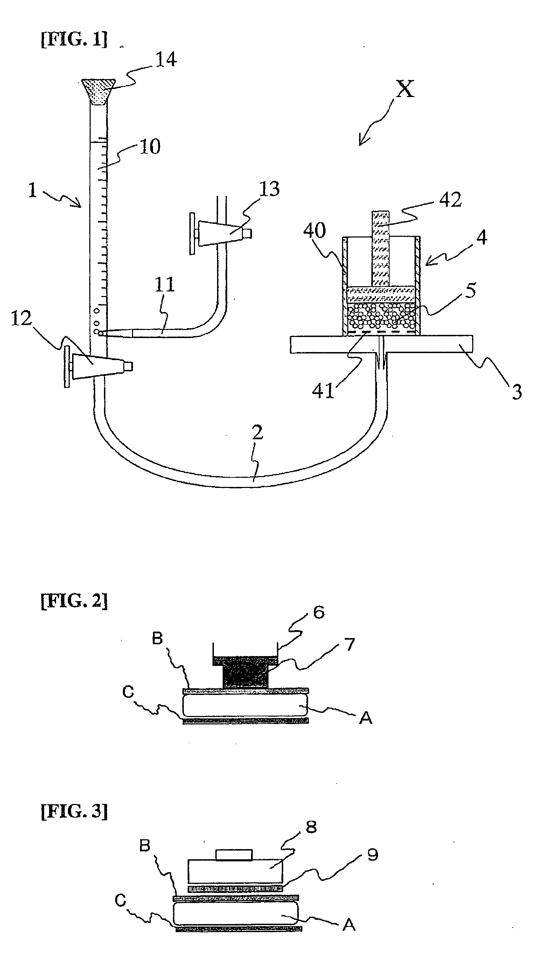

The water-absorption capacity of saline solution of water-absorbent resin under load of 4.14 kPa was measured using a measurement apparatus X of which outline constitution was shown in Figure 1.

The measurement apparatus X shown in Figure 1 comprises a buret section 1, a lead tube 2, a measuring board 3, and a measuring section 4 placed on the measuring board 3. To the buret section 1 are connected a rubber plug 14 at the top of a buret 10, and an air inlet tube 11 and a cock 12 at the bottom portion of the buret 10, and further, the air inlet tube 11 has a cock 13 at the top portion thereof. The lead tube 2 is attached between the buret section 1 and the measuring board 3. The lead tube 2 has a diameter of 6 mm. A hole of a diameter of 2 mm is made at the central section of the measuring board 3, and the lead tube 2 is connected thereto. The measuring section 4 has a cylinder 40, a nylon mesh 41 adhered to the bottom part of the cylinder 40, and a weight 42. The cylinder 40 has an inner diameter of 2.0 cm. The nylon mesh 41 has an opening of 200 mesh (sieve opening: 75 $\mu$m), and is configured so as a predetermined amount of the water-absorbent resin 5 to be evenly spread over the nylon mesh 41. The weight 42 has a diameter of 1.9 cm and a mass of 119.6 g. This weight 42 is placed on the water-absorbent resin 5, so that load of 4.14 kPa can be evenly applied to the water-absorbent resin 5.

In the measurement apparatus X having the configuration above-mentioned, first, the cock 12 and the cock 13 at the buret section 1 are closed, and a physiological saline adjusted to 25°C is poured from the top of the buret 10 and the top of the buret is plugged with the rubber plug 14. Thereafter, the cock 12 and the cock 13 at the buret section 1 are opened. Next, the height of the measuring board 3 is adjusted so that the end of the lead tube 2 in the central section of the measuring board 3 and an air introduction port of the air inlet tube 11 are at the same height.

On the other hand, 0.10 g of the water-absorbent resin 5 is evenly spread over the nylon mesh 41 in the cylinder 40, and the weight 42 is placed on the water-absorbent resin 5. The measuring section 4 is placed so that its center is in alignment with a lead tube port in the central section of the measuring board 3.

The volume reduction of the physiological saline in the buret 10, i.e., the volume of the physiological saline absorbed by the water-absorbent resin 5, Wc (mL), is continuously read off, from a time point where the water-absorbent resin 5 started absorbing water.

In the measurement using the measurement apparatus X, the water-absorption capacity of saline solution under load of the water-absorbent resin 5 after 60 minutes passed from a time point of starting water absorption was calculated by the following formula.

$$\text{Water-Absorption Capacity of Saline Solution Under Load (mL/g)}$$

$$\text{of Water-Absorbent Resin} = \text{Wc (mL)}/0.10 \text{ (g)}$$

< Water Absorption Rate of Saline Solution of Water-Absorbent Resin >

This test was conducted in a room temperature-controlled to 25° ± 1°C. The amount 50 ± 0.1 g of physiological saline was weighed out in a 100 mL beaker, and a magnetic stirrer bar (8 mm$\phi$ × 30 mm, without a ring) was placed therein. The beaker was immersed in a thermostat, of which liquid temperature was controlled to 25° ± 0.2°C. Next, the beaker was placed over the magnetic stirrer so that a vortex was generated in physiological saline at a rotational speed of 600 r/min, the water-absorbent resin was then quickly added in an amount of 2.0 ± 0.002 g to the above beaker, and the time period (seconds) from a point of addition of the water-absorbent resin was added to a point of convergence of the vortex of the liquid surface was measured with a stopwatch , which was defined as a water absorption rate of the water-absorbent resin.

< Mass-Average Particle Size of Water-Absorbent Resin >

An amorphous silica (Sipernat 200, Degussa Japan) was mixed in an amount of 0.5 g as a lubricant with 100 g of a water-absorbent resin.

The above-mentioned water-absorbent resin particles were allowed to pass though a JIS standard sieve having a sieve opening of 250 $\mu$m, and a mass-average particle size was measured using a combination of sieves of (A) in a case where the particles are allowed to pass in an amount of 50% by mass or more, or a combination of sieves of (B) in a case where 50% by mass or more of the particles remain on the sieve.

(A) JIS standard sieves, a sieve having an opening of 425 $\mu$m, a sieve having an opening of 250 $\mu$m, a sieve having an opening of 180 $\mu$m, a sieve having an opening of 150 $\mu$m, a sieve having an opening of 106 $\mu$m, a sieve having an opening of 75 $\mu$m, a sieve having an opening of 45 $\mu$m, and a receiving tray were combined in order from the top.

(B) JIS standard sieves, a sieve having an opening of 850 μm, a sieve having an opening of 600 μa sieve having an opening of 500 μm, a sieve having an opening of 425 μm, a sieve having an opening of 300 μm, a sieve having an opening of 250 um, a sieve having an opening of 150 μm, and a receiving tray were combined in order from the top.

The above-mentioned water-absorbent resin particles were placed on an uppermost sieve of the combined sieves, and shaken for 20 minutes with a rotating and tapping shaker machine to classify the particles.

After classification, the relationships between the opening of the sieve and an integral of a mass percentage of the water-absorbent resin remaining on the sieve were plotted on a logarithmic probability paper by calculating the mass of the water-absorbent resin remaining on each sieve as a mass percentage to an entire amount, and accumulating the mass percentages in order, starting from those having larger particle diameters. A particle diameter corresponding to a 50% by mass cumulative mass percentage is defined as a mass-average particle size by joining the plots on the probability paper in a straight line.

< Water Absorption Capacity of Saline Solution of Water-Absorbent Sheet Composition >

A water-absorbent sheet composition cut into a square of 7 cm each side was used as a sample, and a mass Wd was measured.

Physiological saline was placed in an amount of 500 g in a 500 mL beaker, and a water-absorbent sheet composition sample was added thereto and allowed to stand for 1 hour. The mass We (g) of a JIS standard sieve having a sieve opening of 75 μm was previously measured, and the contents of the above beaker were filtered using this sieve. Excess water content was filtered off by allowing the sieve to stand for 30 minutes in a state that the sieve is slanted at a slant angle of about 30 degrees against the horizon. The mass Wf (g) of the sieve including the sample was measured, and a water absorption capacity of the water-absorbent sheet composition was calculated by the following formula:

$$\text{Water Absorption Capacity (g/m}^2\text{) of Saline Solution of Water-}$$

$$\text{Absorbent Sheet Composition} = [\text{Wf-We-Wd}] \text{ (g)}/0.0049 \text{ (m}^2\text{)}$$

< Evaluation of Properties of Water-Absorbent Sheet Composition >

A water-absorbent sheet composition cut into a rectangular strip of 10 x 30 cm was used as a sample.

In a 10 L vessel were placed 60 g of sodium chloride, 1.8 g of calcium chloride dihydrate, and a proper amount of distilled water to completely dissolve. Next, 15 g of an aqueous 1% by mass poly(oxyethylene) isooctylphenyl ether solution was added thereto, and distilled water was further added to adjust the weight of the overall aqueous solution to 6000 g. Thereafter, the mixed solution was colored with a small amount of Blue No. 1 to prepare a test solution.

On an upper part of a sample (water-absorbent sheet composition) A was placed a polyethylene air-through style porous liquid-permeable sheet B having the same size as the sample ($10 \times 30$ cm) and a basis weight of 21 g/m². In addition, underneath the sample was placed a polyethylene liquid-impermeable sheet C having the same size and basis weight as the sheet B, to prepare a body liquid-absorbent article. A cylindrical cylinder 6 having an inner diameter of 3 cm was placed near the central section of this body liquid-absorbent article, and a 50 mL test solution 7 was supplied thereto at one time. At the same time, a time period until the test solution was completely permeated into the body liquid-absorbent article was measured with a stopwatch, which is referred to as a first permeation rate (sec) (Figure 2). Next, the same procedures were carried out 30 minutes thereafter and 60 minutes thereafter, to measure second and third permeation rates (sec). After 120 minutes from the start of the introduction of the first liquid, 10 cm $\times$ 10 cm filter papers 9, of which mass was previously measured (Wg (g), about 50 g) were stacked near the liquid introductory port, and a 5 kg weight 8 having a size of 10 cm x 10 cm was placed thereon (Figure 3). After 5 minutes of applying a load, the mass (Wh (g)) of the filter papers 9 was measured, and an increased mass was defined as the amount of re-wet (g) as follows.

$$\text{Amount of Re-wet (g)} = \text{Wh} - \text{Wg}$$

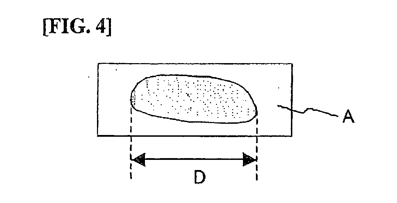

Thereafter, the diffusion length D (cm) of a test solution was measured (Figure 4).

[Examples 1 and 2 and Comparative Examples 1 to 4]

A powder mixture prepared by homogeneously mixing a high-water absorbent resin "AQUA KEEP SA55SX II" (manufactured by Sumitomo Seika Co., Ltd., water retention capacity of saline solution: 35 g/g, water absorption capacity of saline solution under load of 4.14 kPa: 24 mL/g, water absorption rate of saline solution: 42 s, mass-average particle size: 370 $\mu$m) composed of a partially neutralized product of polyacrylic acid (degree of neutralization 75% by mol) and ethylene-vinyl acetate copolymer (melting temperature: 95°C) used as a hot melt adhesive, in a manner that each of the amounts is as shown as the contents listed in Table 1 based on the sheet area, was evenly spread over a hydrophilic nonwoven fabric (back side) composed of a mixture of rayon fibers and polyethylene terephthalate fibers, having a basis weight of 60 g/m$^2$ (rayon fibers/polyethylene terephthalate fibers = 65/35 (mass ratio)). From the upper part, a hydrophilic nonwoven fabric (front side) composed of a mixture of rayon fibers and polyethylene terephthalate fibers, having a basis weight of 60 g/m$^2$ (rayon fibers/polyethylene terephthalate fibers = 65/35 (mass ratio)) was placed thereon, and the layered nonwoven fabrics were formed into a sheet by means of heat-and-pressure, to give a water-absorbent sheet composition.

[Comparative Example 5]

The same procedures as in Example 1 except that a hydrophobic nonwoven fabric (hydrophobic polyethylene terephthalate (100%) having a basis weight of 40g/m$^2$) was used in place of the hydrophilic nonwoven fabric as a nonwoven fabric (back side) to which a water-absorbent resin and a hot melt adhesive were spread in Example 1 was used to form a sheet, to obtain a water-absorbent sheet composition.

The properties of the water-absorbent sheet compositions obtained in Examples 1 and 2 and Comparative Examples 1 to 5 were measured according to the above methods. The determination results for the presence or absence of liquid leakage upon introduction of a test solution, the permeation rates, the amount of re-wet, and the diffusion length, and the presence or absence of scattering of a water-absorbent resin are shown in Table 1.

[Table 1]

| | Kinds of Nonwoven Fabric (Front Side/ Back Side) | Content X of Water-Absorbent Resin [g/m²] | Content Y of Adhesive [g/m²] | Y/X | Thickness [mm] | Water Absorption Capacity of Physiological Saline [g/m²] | Liquid Leakage from Body liquid Absorbent Article | Permeation Rate [sec] | | | | Amount of Re-wet [g] | Diffusion Length [cm] | Scattering of Water-Absorbent Resin |
|---|---|---|---|---|---|---|---|---|---|---|---|---|---|---|
| | | | | | | | | 1 | 2 | 3 | Total | | | |
| Ex.1 | Hydrophilic/ Hydrophilic | 270 | 54 | 0.20 | 1.1 | 11000 | Not observed | 44 | 21 | 21 | 86 | 2.7 | 26 | Not observed |
| Ex.2 | Hydrophilic/ Hydrophilic | 330 | 53 | 0.16 | 1.4 | 14000 | Not observed | 58 | 33 | 19 | 110 | 0.2 | 29 | Not observed |
| Comp. Ex.1 | Hydrophilic/Hydrophilic | 150 | 30 | 0.20 | 1.0 | 5000 | Not observed | 36 | 20 | 20 | 76 | 18.7 | 30 | Not observed |
| Comp. Ex.2 | Hydrophlic/ Hydrophilic | 430 | 69 | 0.16 | 1.5 | 17000 | Not observed | 109 | 63 | 34 | 206 | 0.2 | 22 | Not observed |
| Comp. Ex. 3 | Hydrophilic/ Hydrophilic | 270 | 21 | 0.08 | 1.1 | 12000 | Not observed | 45 | 31 | 22 | 98 | 1.5 | 24 | Observed |
| Comp. Ex. 4 | Hydrophilic/ Hydrophilic | 300 | 159 | 0.53 | 1.0 | 4500 | Not observed | 112 | 70 | 28 | 210 | 10.9 | 30 | Not observed |
| Comp. Ex. 5 | Hydrophilic/ Hydrophobic | 270 | 54 | 0.20 | 1.4 | 10000 | Observed | Undeterminable | | | | | | Not observed |

It can be seen from the above results that the water-absorbent sheet compositions of Examples 1 and 2 have fast permeation rates, small amount of re-wet, and excellent diffusion of the liquid, as compared to those of Comparative Examples 1 to 4. In addition, in the water-absorbent sheet composition of Comparative Example 5 where a hydrophobic nonwoven fabric was used on the back side, a test solution is not absorbed in a water-absorbent sheet during the introduction of a test liquid, and a liquid leakage occurs from the water-absorbent sheet. Therefore, comparative evaluations could not be made.

INDUSTRIAL APPLICABILITY

The water-absorbent sheet composition of the present invention can be suitably used in hygienic material fields, agricultural fields, construction material fields, and the like, among which the water-absorbent sheet composition can be suitably used for disposable diapers.

EXPLANATION OF NUMERICAL SYMBOLS

X       measurement apparatus
1       buret section
10      buret
11      air inlet tube
12      cock
13      cock
14      rubber plug
2       lead tube
3       measuring board
4       measuring section
40      cylinder
41      nylon mesh
42      weight
5       water-absorbent resin
6       cylindrical cylinder
7       test solution
8       weight
9       filter paper
A       sample (water-absorbent sheet composition)
B       liquid-permeable sheet
C       liquid-impermeable sheet
D       diffusion length

**Claims**

1.   A water-absorbent sheet composition comprising a structure in which a water-absorbent resin and a hot melt adhesive are sandwiched with two or more sheets of hydrophilic nonwoven fabrics, wherein the hydrophilic nonwoven fabrics have a basis weight of 25 g/m$^2$ or more, and wherein the water-absorbent resin is contained in an amount of from 200 to 400 g/m$^2$, and wherein the hot melt adhesive is contained in an amount of from 0.10 to 0.50 times that of the amount of the water-absorbent resin contained (mass basis),
     wherein the hydrophilic nonwoven fabric is made of rayon fibers or a mixture of two or more kind of fibers including rayon fibers, and
     wherein the water absorbent resin is uniformly immobilized on the hydrophilic nonwoven fabric.

2.   The water-absorbent sheet composition according to claim 1, wherein the hydrophilic nonwoven fabric is made of rayon fibers and, optionally, at least one member selected from the group consisting polyolefin fibers, polyester fibers and mixtures thereof.

3.   The water-absorbent sheet composition according to claim 1 or 2, wherein the water-absorbent resin has a water retention capacity of saline solution of 25 g/g or more.

**4.** The water-absorbent sheet composition according to any one of claims 1 to 3, wherein the hot melt adhesive is at least one member selected from ethylene-vinyl acetate copolymer adhesives and stryrenic elastomer adhesives.

**5.** The water-absorbent sheet composition according to any one of claims 1 to 4, wherein the water-absorbent sheet composition has a thickness of 5 mm or less on a dry basis.

**6.** The water-absorbent sheet composition according to any one of claims 1 to 5, wherein the water-absorbent sheet composition has a water absorption capacity of saline solution of 2000 $g/m^2$ or more.

**7.** An absorbent article comprising the water-absorbent sheet composition as defined in any one of claims 1 to 6, sandwiched between a liquid-permeable sheet and a liquid-impermeable sheet.


**Patentansprüche**

**1.** Wasserabsorbierende Bahnzusammensetzung, die eine Struktur umfasst, in der ein wasserabsorbierendes Harz und ein Heißschmelzklebemittel sandwichartig zwischen zwei oder mehrere Bahnen von hydrophilem Vliesmaterial eingebracht sind, worin das hydrophile Vliesmaterial ein Basisgewicht von 25 $g/m^2$ oder mehr aufweist, und worin das wasserabsorbierende Harz in einer Menge von 200 bis 400 $g/m^2$ enthalten ist, und worin das Heißschmelzklebemittel in einer Menge des bis 0,10 bis 0,50-fachen der Menge des enthaltenen wasserabsorbierenden Harzes (Massenbasis) enthalten ist,
worin das hydrophile Vliesmaterial aus Rayon-Fasern oder einer Mischung von zwei oder mehreren Sorten von Fasern, einschließlich Rayon-Fasern, hergestellt ist, und
worin das wasserabsorbierende Harz gleichförmig auf dem hydrophilen Vliesmaterial immobilisiert ist.

**2.** Wasserabsorbierende Bahnzusammensetzung gemäß Anspruch 1, worin das hydrophile Vliesmaterial aus Rayon-Fasern und optional mindestens einem Element, ausgewählt aus der Gruppe bestehend aus Polyolefin-Fasern, Polyesterfasern und Mischungen daraus, hergestellt ist.

**3.** Wasserabsorbierende Bahnzusammensetzung gemäß Anspruch 1 oder 2, worin das wasserabsorbierende Harz eine Wasserhaltekapazität von Kochsalzlösung von 25 g/g oder mehr aufweist.

**4.** Wasserabsorbierende Bahnzusammensetzung gemäß irgendeinem der Ansprüche 1 bis 3, worin das Heißschmelzklebemittel mindestens ein Element, ausgewählt aus Ethylen-Vinylacetat-Copolymer-Klebemittel und Styrolelastomer-Klebemittel, ist.

**5.** Wasserabsorbierende Bahnzusammensetzung gemäß irgendeinem der Ansprüche 1 bis 4, worin die wasserabsorbierende Bahnzusammensetzung eine Dicke von 5 mm oder mehr auf Trockenbasis aufweist.

**6.** Wasserabsorbierende Bahnzusammensetzung gemäß irgendeinem der Ansprüche 1 bis 5, worin die wasserabsorbierende Bahnzusammensetzung eine Wasserabsorptionskapazität von Kochsalzlösung von 2000 $g/m^2$ oder mehr aufweist.

**7.** Absorbierendes Erzeugnis, das die wasserabsorbierende Bahnzusammensetzung, wie in irgendeinem der Ansprüche 1 bis 6 definiert, umfasst, die sandwichartig zwischen eine flüssigkeitsdurchlässige Bahn und eine flüssigkeitsundurchlässige Bahn eingebracht ist.


**Revendications**

**1.** Composition de feuille absorbant l'eau, comprenant une structure dans laquelle une résine absorbant l'eau et un adhésif thermofusible sont pris en tenailles avec deux ou plusieurs feuilles de tissus non tissés hydrophiles, dans laquelle les tissus non tissés hydrophiles ont un grammage supérieur ou égal à 25 $g/m^2$, et dans laquelle la résine absorbant l'eau est contenue en une quantité allant de 200 et 400 g/m2, et dans laquelle l'adhésif thermofusible est contenu en une quantité allant de 0,10 et 0,50 fois la quantité de la résine absorbant l'eau contenue (base de masse),
dans laquelle le tissu non tissé hydrophile est réalisé en fibres de rayonne ou en un mélange de deux ou de plusieurs types de fibres comportant des fibres de rayonne, et

dans laquelle la résine absorbant l'eau est immobilisée de manière uniforme sur le tissu non tissé hydrophile.

2.  Composition de feuille absorbant l'eau selon la revendication 1, dans laquelle le tissu non tissé hydrophile est réalisé en fibres de rayonne et, éventuellement, en au moins un élément choisi dans le groupe constitué de fibres de polyoléfines, de fibres de polyester et de mélanges de celles-ci.

3.  Composition de feuille absorbant l'eau selon la revendication 1 ou 2, dans laquelle la résine absorbant l'eau présente une capacité de rétention d'eau d'une solution saline supérieure ou égale à 25 g/g.

4.  Composition de feuille absorbant l'eau selon l'une quelconque des revendications 1 à 3, dans laquelle l'adhésif thermofusible est au moins un élément choisi parmi des adhésifs à base de copolymère d'éthylène-acétate de vinyle et des adhésifs à base d'élastomère styrénique.

5.  Composition de feuille absorbant l'eau selon l'une quelconque des revendications 1 à 4, dans laquelle la composition de feuille absorbant l'eau présente une épaisseur inférieure ou égale à 5 mm sur une base sèche.

6.  Composition de feuille absorbant l'eau selon l'une quelconque des revendications 1 à 5, dans laquelle la composition de feuille absorbant l'eau présente une capacité d'absorption d'eau d'une solution saline supérieure ou égale à 2000 g/m$^2$.

7.  Article absorbant comprenant la composition de feuille absorbant l'eau telle que définie dans l'une quelconque des revendications 1 à 6, prise en tenailles entre une feuille perméable aux liquides et une feuille imperméable aux liquides.

**[FIG. 1]**

**[FIG. 2]**

**[FIG. 3]**

[FIG. 4]

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2001252307 A
- JP HEI06057010 B
- JP HEI10118114 B
- JP 2000238161 A

- JP 2003011118 A
- JP HEI02048944 B
- WO 2004016425 A
- US 2002115969 A